# FASCICULE DE BREVET EUROPEEN

(11) **EP 0 614 663 B1**
(45) Date de publication et mention de la délivrance du brevet: **08.09.1999**
(21) Numéro de dépôt: 94400504.0
(22) Date de dépôt: 08.03.1994
(51) Int. Cl.: A61K 31/445, A61K 31/38, A61K 31/135

(54) **Composition pharmaceutique anesthésique comprenant un anesthésique général et de la sélégiline**
Pharmazeutische Zusammensetzung, die ein allgemeines Anästhetikum ung Selegilin enthält
Pharmaceutical composition containing a general anaesthetic and selegiline

(30) Priorité: 09.03.1993 FR 9302713; 22.12.1993 FR 9315496
(43) Date de publication de la demande: 14.09.1994
(73) Titulaire: SANOFI SANTE NUTRITION ANIMALE, 33500 Libourne (FR)
(72) Inventeur: Ovaert, Patricia, F-33200 Bordeaux (FR); Boivin, Eliane, F-33310 Pompignac (FR)
(74) Mandataire: Le Guen, Gérard

(56) Documents cités:
- US-A- 3 879 535
- US-A- 3 896 221
- US-A- 4 017 619

## Description

La présente invention concerne des compositions pharmaceutiques pour réaliser une anesthésie générale chez les mammifères et notamment chez les animaux de compagnie.

En médecine vétérinaire, lors des opérations de courte durée, inférieure à 1 heure, on administre fréquemment à l'animal de la kétamine ou de la tilétamine, médicaments qui induisent l'anesthésie peu de temps après leur injection par voie intraveineuse ou intramusculaire, et qui conduisent à un réveil rapide.

Ces composés ne sont pas sans effets secondaires et on a proposé de les associer à des benzodiazépines, à la xylazine ou à l'acépromazine comme mentionné dans JAVMA 180(12), 1462-1471 (1982).

Mais ces associations prolongent la durée de l'anesthésie et sont encore souvent à l'origine d'hallucinations et d'une hyperexcitabilité à tout stimulus externe pendant la phase d'induction et celle de réveil.

L'administration des compositions selon l'invention n'induit pas ou peu d'hallucinations et de mouvements incoordonnés pendant les phases d'induction et de réveil ; en outre, la rigidité musculaire observée durant l'anesthésie à la kétamine ou à d'autres dérivés de la phencyclidine, à l'origine de convulsions, de spasmes et de crises épileptiformes, diminue sensiblement pour un degré d'analgésie équivalent ; enfin, on ne constate pas d'aggravation des effets cardiovasculaires connus de la kétamine, notamment de l'effet hypertenseur et tachycardisant.

Les compositions selon l'invention comprennent un anesthésique général de type de phencyclidine, tel que la kétamine ou la tilétamine et leurs sels pharmaceutiquement acceptables ainsi que de la sélégiline ou un de ses sels pharmaceutiquement acceptables, associés dans une forme pharmaceutique unique pour une administration simultanée ou se présentant séparément pour des administrations successives rapprochées. Dans ce dernier cas, la sélégiline a un rôle de pré-anesthésique ou agent de contention.

La tilétamine est la 2-(éthylamino)-2-(2-thiényl)-cyclohexanone. La kétamine est la (±)-2-(2-chlorophényl)-2-méthylaminocyclohexanone. La sélégiline est la (-)-N,α-diméthyl-N-2-propynylphénéthylamine.

Les compositions peuvent se présenter sous forme de poudres, lyophilisées ou non, à diluer au moment de l'emploi dans un véhicule pour injection, aqueux ou huileux ou sous forme de suspensions ou de solutions aqueuses ou huileuses injectables dans lesquelles le principe actif peut être associé aux adjuvants classiques.

La dose unitaire de composition selon l'invention contiendra la dose d'anesthésique habituelle du composé phéncyclidine associée à 0,015 à 1,25 équivalent molaire de sélégiline, de préférence 0,050 à 1,0, avantageusement de 0,25 à 1,0.

Notamment chez le chien et le chat, on pourra administrer par voie intraveineuse de 5 à 8 mg/kg de kétamine avec 0,1 mg/kg à 5 mg/kg, notamment 0,25 mg/kg à 4 mg/kg, et avantageusement de 1 à 4 mg/kg de sélégiline, HCl en solution aqueuse pour obtenir une anesthésie d'une demi-heure environ ; les injections seront en général de 0,05 à 0,5 ml/kg, et notamment de 0,125 à 0,5 ml/kg de poids corporel.

On sait que la molécule de sélégiline comporte un carbone asymétrique et que seul cet isomère lévogyre est un inhibiteur de la monoamine oxydase de type B ; par conséquent, les compositions selon l'invention peuvent aussi contenir l'isomère d, ou le mélange racémique de sélégiline (dénommée aussi déprényl) dans les proportions requises pour l'isomère l (supposant que l'isomère d est peu ou pas actif aussi dans le cadre de la présente invention).

Lorsque l'anesthésique et la sélégiline sont conditionnées séparément, on peut mélanger les 2 solutions extemporanément au moment de l'injection ou on peut administrer la sélégiline de 5 à 20 minutes avant, notamment par voie sous-cutanée.

### Etude 1 : solutions séparées de sélégiline et de kétamine.

Dans ce qui suit, on décrit des observations cliniques effectuées lors de l'anesthésie générale de chiens Beagle auxquels ont été injectés par voie intraveineuse, environ 2 à 3 ml de solution aqueuse de chlorhydrate de kétamine et de chlorhydrate de sélégiline dans les proportions mentionnées dans le tableau I suivant. Chaque solution a été administrée 2 fois à 2 chiens à 4 jours d'intervalle ; on avait préalablement administré aux mêmes animaux, 5 jours avant, de la kétamine seule.
Dans le tableau I, on indique en colonne :
(A) : le temps d'induction de l'anesthésie (min)
(B) : la durée de l'anesthésie (min)
(C) : le temps écoulé entre l'injection et l'apparition d'une locomotion spontanée (min)
(D) : le temps écoulé entre l'injection et l'observation d'un comportement normal (min)
(E) : le degré d'analgésie lors du pincement d'un lobe auriculaire par un clamp chirurgical (score de 0 à 3, le score 3 correspondant à un bon degré d'analgésie).
(F) : le degré de relaxation musculaire lors de la mobilisation de membres de l'animal en décubitus (score de 0 à 3, le score 3 signifiant une grande relaxation).
(G) : l'importance des mouvements spontanés de type spasme (score 1), crise épileptiforme (score 2) ou convulsion (score 3).

Pour les 3 dernières observations, le score indiqué est la somme des neuf valeurs relevées toutes les 5 minutes pendant 40 minutes (valeur moyenne pour 2 chiens).

Les résultats montrent que la composition anesthésique selon l'invention, utilisée en solutions séparées, permet une anesthésie de courte durée en évitant les épisodes convulsifs ainsi qu'une mauvaise myorelaxation observée avec la kétamine seule.

**TABLEAU I**

| Kétamine HCl mg/kg | sélégiline HCl mg/kg | A | B | C | D | E | F | G |
|---|---|---|---|---|---|---|---|---|
| 8 | 0 | 0,42 | 16,2 | 20,3 | 40,3 | 14,7 | 2,5 | 8,2 |
| 8 | 0,125 | 0,42 | 15,5 | 18 | 32 | 11,5 | 2,5 | 2 |
| 8 | 0,500 | 0,38 | 17,5 | 20 | 35,5 | 15 | 8,0 | 3,5 |
| 8 | 1,0 | 0,35 | 15 | 19,5 | 31,5 | 13,5 | 6,0 | 0,5 |

### Etude 2 : solution unique de kétamine/sélégiline

La composition utilisée comprend :

| | |
|---|---|
| Kétamine, HCl | 50,0 mg |
| Sélégiline, HCl | 10,0 mg |
| Excipient (eau) q.s.p. | 1,0 ml |

Cette étude est réalisée sur 6 chiens Beagle (3 mâles et 3 femelles).
L'injection est en IV et de 1,6 ml/kg.

Le protocole et les critères d'évaluation sont identiques à l'Etude 1. Les résultats sont regroupés dans le tableau II ci-dessous. Les valeurs indiquées sont des moyennes sur 6 Beagles.

**TABLEAU II**

| **Paramètres** | **A** | **B** | **C** | **D** | **E** | **F** | **G** |
|---|---|---|---|---|---|---|---|
| Valeur moyenne 6 beagles | 0,45 ± 0,06 | 19,1 ± 4,6 | 26,6 ± 5,06 | 39,6 ± 7,16 | - | 6,1 | 0,68 |

Cette étude confirme les résultats obtenus dans l'étude 1 mais avec une solution unique de la composition selon l'invention.

### Etude 3 : solution de sélégiline seule en SC

La concentration des solutions varie de 1 mg/kg à 3 mg/kg. L'injection de sélégiline HCl a été effectuée en sous-cutanée, 20 minutes avant l'injection en IV de la kétamine ou de la tilétamine. Chaque dose est testée sur 4 chats (2 mâles et 2 femelles - race européenne).

L'électromyogramme et l'électroencéphalogramme permettent respectivement d'évaluer les paramètres de myorelaxation, d'eidolies hallucinosiques récurrentes qui sont caractérisées par des polypointes de 80 à 150 µV et de 18 à 20 Hz sur l'enregistrement EEG (electroencéphalogramme).

Les résultats sont rassemblées dans le tableau III ci-dessous.

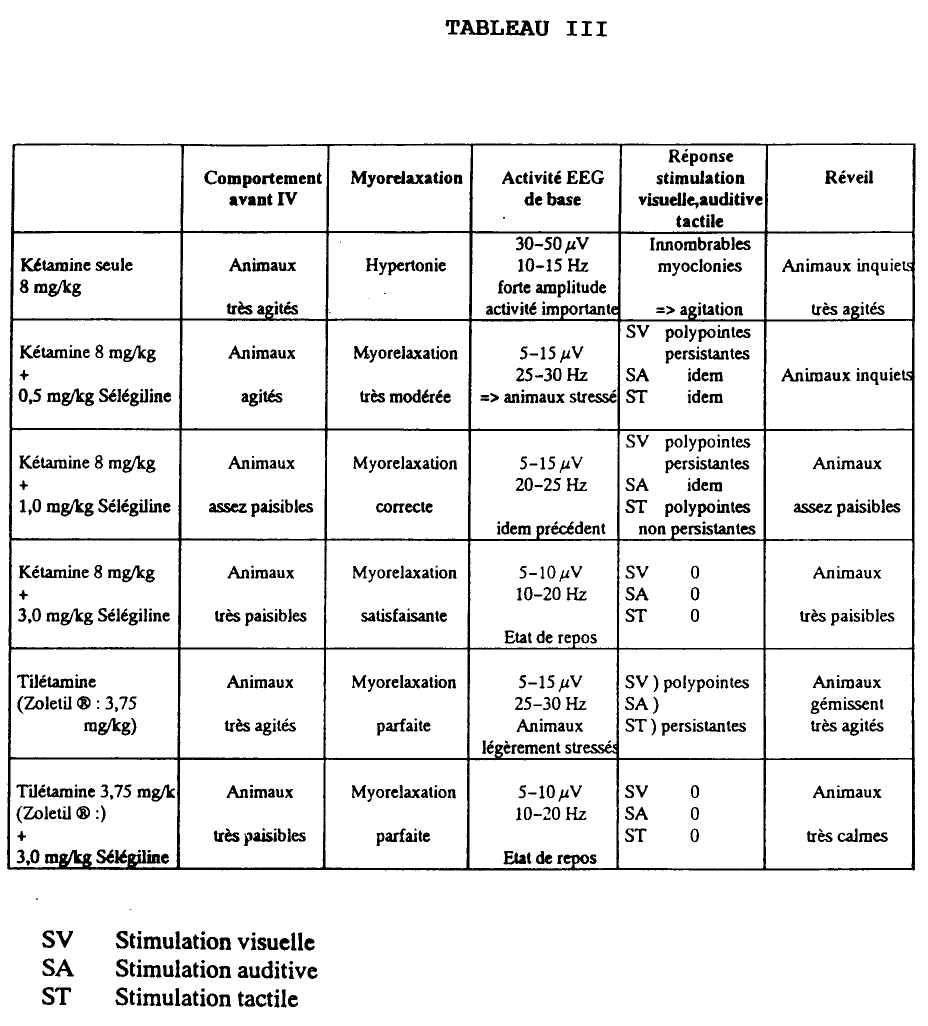

L'étude 3 a mis en évidence que l'utilisation de la sélégiline prévient des eidolies hallucinosiques récurrentes après l'anesthésie à la kétamine ou la tilétamine et facilite la manipulation des animaux avant injection de ces molécules.

## Revendications

1. Composition pharmaceutique vétérinaire pour induire une anesthésie générale, caractérisée en ce qu'elle comprend avec les véhicules usuels un anesthésique général de type phéncyclidine, tel que la kétamine ou la tilétamine ou l'un de leurs sels pharmaceutiquement acceptables ainsi que de 0,015 à 1,25 équivalent molaire de sélégiline ou de l'un de ses sels pharmaceutiquement acceptables, associés dans une forme pharmaceutique unique ou présentés séparément.

2. Composition selon la revendication 1, caractérisée en ce qu'elle comprend le composé de type phéncyclidine ou l'un de ses sels, ainsi que de 0,050 à 1,0 équivalent molaire de sélégiline ou de l'un de ses sels.

3. Composition selon la revendication 1, caractérisée en ce qu'elle comprend de la kétamine ou l'un de ses sels et de 0,25 à 1,0 équivalent molaires de selégiline, ou de l'un de ses sels, par rapport à la kétamine.

4. Composition selon l'une des revendications 1 à 3, caractérisée en ce qu'une dose unitaire comprend la quantité nécessaire pour administrer de 1 à 4 mg/kg de sélégiline, HCl à un chien ou à un chat.

5. Utilisation de sélégiline ou d'un de ses sels pharmaceutiquement acceptable pour la fabrication d'un médicament vétérinaire prévenant les effets secondaires de l'anesthésie à la kétamine ou la tilétamine, ou leurs sels pharmaceutiquement acceptables, notamment l'hypertonie musculaire, les crises épileptiformes et les eidolies hallucinosiques récurrentes.

## Claims

1. Veterinary Pharmaceutical compositions for inducing general anaesthesia, characterised in that it comprises, together with conventional carriers, a general anaesthetic of the phencyclidine type such as ketamine or tiletamine or one of the pharmaceutically acceptable salts thereof as well as 0.015 to 1.25 molar equivalents of selegiline or one of the pharmaceutically acceptable salts thereof, combined in a single pharmaceutical preparation or presented separately.

2. Composition according to claim 1, characterised in that it comprises the compound of the phencyclidine type or one Of the salts thereof, as well as 0.050 to 1.0 molar equivalents of selegiline or one of the pharmaceutically acceptable salts thereof.

3. Composition according to claim 1, characterised in that it comprises ketamine or one of the salts thereof and 0.25 to 1.0 molar equivalents, based on the ketamine, of selegiline or one of the salts thereof.

4. Composition according to one of claims 1 to 3, characterised in that a single dose contains the quantity needed to administer from 1 to 4 mg/kg of selegiline HCl to a dog or cat.

5. Use of selegiline or one of the pharmaceutically acceptable salts thereof for the production of a veterinary medicament which prevents the side effects of anaesthesia with ketamine or tiletamine or with the pharmaceutically acceptable salts thereof, notably muscular hypertonia, epileptiform seizures and recurrent hallucinations.

## Patentansprüche

1. Veterinär-pharmazeutische Zubereitung zur Induktion einer Allgemeinanästhesie, dadurch gekennzeichnet, daß sie zusammen mit üblichen Trägermaterialien ein Allgemeinanästhetikum des Typs Phencyclidin, wie Ketamin oder Tiletamin, oder eines ihrer pharmazeutisch annehmbaren Sage, sowie 0,015 bis 1.25 Moläquivalente Selegilin oder eines seiner pharmazeutisch annehmbaren Salze in einer einzigen pharmazeutischen Darreichungsform oder getrennten Darreichungsform umfaßt.

2. Zubereitung nach Anspruch 1, dadurch gekennzeichnet, daß sie die Verbindung vom Typ Phencyclidin oder eines seiner Salze sowie 0.050 bis 1,0 Moläquivalente Selegilin oder eines seiner Salze umfaßt.

3. Zubereitung nach Anspruch 1, dadurch gekennzeichnet, daß sie Ketamin oder eines seiner Salze und 0,25 bis 1,0 Moläquivalente Selegilin oder einen seiner Salze, bezogen auf das Ketamin, umfaßt.

4. Zubereitung nach einem der Ansprüche 1 bis 3, dadurch gekennzeichnet, daß eine Einheitsdosis die Menge umfaßt, die dazu notwendig ist, 1 bis 4 mg/kg Selegilin, HCl an einen Hund oder eine Katze zu verabreichen.

5. Verwendung von Selegilin oder eines seiner pharmazeutisch annehmbaren Salze für die Herstellung eines veterinärmedizinischen Arzneimittels zur Vorbeugung von Nebeneffekten der Anästhesie mit Ketamin oder Tiletamin oder ihrer pharmazeutisch annehmbaren Salze, insbesondere der Muskelhypertonie, von epileptiformen Krisen und wiederholten halluzinösen Eidolien.
